(19) Europäisches Patentamt
European Patent Office
Office européen des brevets

(11) EP 3 524 067 B1

(12) **EUROPEAN PATENT SPECIFICATION**

(45) Date of publication and mention of the grant of the patent:
**02.04.2025 Bulletin 2025/14**

(21) Application number: **18155669.7**

(22) Date of filing: **08.02.2018**

(51) International Patent Classification (IPC):
***A23L 33/00*** (2016.01) ***C07H 1/08*** (2006.01)
***C07H 3/06*** (2006.01) ***A23L 29/30*** (2016.01)
***A23L 33/21*** (2016.01) ***C07H 3/02*** (2006.01)
***C07H 13/04*** (2006.01)

(52) Cooperative Patent Classification (CPC):
**A23L 33/40; A23L 29/30; A23L 33/21; C07H 1/08; C07H 3/02; C07H 3/06; C07H 13/04**

(54) **SPRAY-DRIED MIXTURE OF HUMAN MILK OLIGOSACCHARIDES**

SPRÜHGETROCKNETE MISCHUNG HUMANER MILCH-OLIGOSACCHARIDE

MÉLANGE SÉCHANT PAR PULVÉRISATION D'OLIGOSACCHARIDES DE LAIT HUMAIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**14.08.2019 Bulletin 2019/33**

(73) Proprietor: **Chr. Hansen HMO GmbH**
**53619 Rheinbreitbach (DE)**

(72) Inventor:
- **The inventor has waived his right to be thus mentioned.**

(56) References cited:
**EP-A1- 2 896 628** **EP-A1- 3 486 326**
**EP-A1- 3 494 804** **EP-A1- 3 494 805**
**EP-A1- 3 494 806** **EP-A1- 3 494 807**
**WO-A1-2015/049331** **US-A1- 2015 183 814**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 524 067 B1

## Description

The present invention relates to spray-dried powders containing a mixture of structurally distinct human milk oligosaccharides and at least one monosaccharide selected from the group consisting of L-fucose and N-acetylneuraminic acid, and to their use for manufacturing nutritional compositions.

## Background

Human breast milk contains substantial amounts of carbohydrates. The carbohydrates that are present in human breast milk include monosaccharides such as L-fucose and N-acetylneuraminic acid, the disaccharide lactose, and up to 20 g/l oligosaccharides, the so-called "human milk oligosaccharides (HMOs)". HMOs represent the third most abundant constituent of human breast milk. It is presumed that more than 150 structurally distinct oligosaccharides are present in human milk. Selected HMOs are shown in Table 1. Each of about 10 to 13 of these HMOs are present in human milk in a concentration of between several hundred milligrams to grams per liter (Thurl et al., (2017), Nutrition Reviews 75(11) 920-933). Among the HMOs, neutral HMOs are known as well as acidic HMOs which contain at least one N-acetylneuraminic acid (NeuAc) moiety. The structural complexity and abundance of these oligosaccharides is unique for human milk and has not been found in the milk of other mammals such as - for example - domesticated dairy animals.

Since HMOs are not digested by humans, the physiological role of these saccharides has been under investigation for several decades. The prebiotic effect of HMOs was discovered more than 100 years ago. Upon consumption, HMOs are able to modulate the composition of the human gut microbiome by supporting growth of beneficial bacteria.

Table 1: Structures of notable oligosaccharides in human breast milk.

| Name | Chemical structure |
|---|---|
| 2'-FL | **Fuc**(α1,2)**Gal**(β1,4)**Glc** |
| 3-FL | **Gal**(β1,4)**(Fuc**(α1,3))**Glc** |
| DFL | **Fuc**(α1,2)**Gal**(β1,4)**Glc**<br>(α1,2)\|<br>**Fuc** |
| LNT | **Gal**(β1,3)**GlcNAc**(β1,3)**Gal**(β1,**4)Glc** |
| LN*n*T | **Gal**(β1,4)**GlcNAc**(β1,3)**Gal**(β1,**4)Glc** |
| LNFP-I | **Fuc**(α1,2)**Gal**(β1,3)**GlcNAc**(β1,3)**Gal**(β1,4)**Glc** |
| LNFP-II | **Gal**(β1,3)**(Fuc**(α1,4)**)GlcNAc**(β1,3)**Gal**(β1,4)**Glc** |
| LNFP-III | **Gal**(β1,4)**(Fuc**(α1,3)**)GlcNAc**(β1,3)**Gal**(β1,4)**Glc** |
| LNFP-V | **Gal**(β1,3)**GlcNAc**(β1,3)**Gal**(β1,4)**(Fuc**(α1,3)**)Glc** |
| 3'-SL | **NeuAc**(α2,3)**Gal**(β1,4)**Glc** |
| 6'-SL | **NeuAc**(α2,6)**Gal**(β1,4)**Glc** |
| LSTa | **NeuAc**(α2,3)**Gal**(β1,3)**GlcNAc**(β1,3)**Gal**(β1,4)**Glc** |
| LSTb | **NeuAc** \|(α2,6) **Gal**(β1,3)**GlcNAc**(β1,3)**Gal**(β1,4)**Glc** |
| LSTc | **NeuAc**(α2,6)**Gal**(β1,4)**GlcNAc**(β1,3)**Gal**(β1,4)**Glc** |
| LNDFH-I | **Fuc**(α1,2)**Gal**(β1,3)**GlcNAc**(β1,3)**Gal**(β1,4)**Glc** \|(α1,4) **Fuc** |
| LNDFH-II | **Gal**(β1,3)**GlcNAc**(β1,3)**Gal**(β1,4)**Glc**<br>\|(α1,4) (α1,3)\|<br>**Fuc** **Fuc** |
| LNDFH-III | **Gal**(β1,3)**GlcNAc**(β1,3)**Gal**(β1,4)**Glc**<br>\|(α1,3) (α1,3)\|<br>**Fuc** **Fuc** |

(continued)

<table>
<tr><th>Name</th><th>Chemical structure</th></tr>
<tr><td>F-LSTa</td><td>NeuAc(α2,3)Gal(β1,3)GlcNAc(β1,3)Gal(β1,4)Glc<br>| (α1,4)<br>Fuc</td></tr>
<tr><td>F-LSTb</td><td>NeuAc<br>| (α2,6)<br>Fuc(α1,2)Gal(β1,3)GlcNAc(β1,3)Gal(β1,4)Glc</td></tr>
<tr><td>F-LSTc</td><td>NeuAc(α2,6)Gal(β1,4)GlcNAc(β1,3)Gal(β1,4)Glc<br>(α1,3) |<br>Fuc</td></tr>
<tr><td>FSL</td><td>NeuAc(α2,3)Gal(β1,4)Glc (α1, 3) | Fuc</td></tr>
<tr><td>DS-LNT</td><td>NeuAc<br>| (α2,6)<br>NeuAc(α2,3)Gal(β1,3)GlcNAc(β1,3)Gal(β1,4)Glc</td></tr>
<tr><td>FDS-LNT-I</td><td>NeuAc<br>| (α2,6)<br>NeuAc(α2,3)Gal(β1,4)GlcNAc(β1,3)Gal(β1,4)Glc</td></tr>
<tr><td>FDS-LNT-II</td><td>NeuAc<br>| (α2,6)<br>NeuAc(α2,3)Gal(β1,4)GlcNAc(β1,3)Gal(β1,4)Glc<br>(α1,3) |<br>Fuc</td></tr>
</table>

Several other functional effects of HMOs were revealed in the last years, especially their effect on the development of neonates. HMOs are known to act as decoys to reduce the risk of infections by bacterial and viral pathogens which adhere to human cells by binding to these cells' surface glycoproteins. Additionally, various HMOs possess an anti-inflammatory effect and act as immunomodulators. Hence, it was proposed that HMOs reduce the risks of developing food allergies. A positive effect of sialylated HMOs on the development of a neonate's central nervous system is also intensely discussed (reviewed in "Prebiotics and Probiotics in Human Milk, Origins and functions of milk-borne oligosaccharides and bacteria", Academic Press (2017) editors: McGuire M., McGuire M., and Bode L.).

To take advantage of the beneficial effects of HMOs, efforts are made in adding individual HMOs to nutritional composition, in particular to infant formula. However, supplementing nutritional compositions with a combination of different HMOs would be better, because such compositions more closely resemble the natural source of HMOs, i.e. human milk, and are more likely to possess better effects on a person's health and development than compositions containing only a single species of the HMOs.

The limited supply of HMOs for supplementing nutritional compositions led to the development of procedures for chemical syntheses of HMOs. The drawbacks in such chemical syntheses lead to biocatalytic approaches wherein HMOs are synthesized in vitro using purified enzymes such as glycosyltransferases. Today, individual HMOs are produced in an industrial scale using fermentation of genetically-engineered microbial cells (WO 2015/150328 A1, WO 2017/043382 A1, WO 2010/070104 A1, WO 2012/097950 A1). The HMOs are synthesized by genetically-engineered microbial cells and can be recovered from the fermentation medium and/or cell lysate to obtain a substantially pure preparation of the HMO.

During their recovery from a fermentation broth, HMOs are usually present in form of a liquid process stream, e.g. an aqueous solution which contains the HMO of interest and which may also contain undesired HMOs which are by-products that were generated during the fermentative production of the desired HMO. Along with the recovery of the desired HMO, i.e. the HMO of interest, its concentration in the process stream and its purity is increased. However, an aqueous solution containing HMOs is vulnerable to bacterial or fungal contamination. Therefore, it is preferred to provide the desired HMOs as a dry or solid product which contains a low amount of water. Growth of microbial organisms is hardly possible on/in such a solid product.

Typically, a saccharide is obtained in solid form by crystallization. Crystallization of individual HMOs from an aqueous solution has been described for 3-fucosyllactose (WO 2014/075680 A), for 2'-fucosyllactose (WO 2011/150939 A), di-fucosyllactose (WO 2016/086947 A), lacto-N-tetraose (WO 2017/101953 A), lacto-N-neotetraose (WO 2014/094783 A). Crystallization of HMOs involves the use of organic solvents such as alcohols, mainly ethanol or methanol, or organic acids such as glacial acetic acid. However, the use of alcohols, especially methanol, for crystallizing HMOs at the end of the recovery process is inappropriate if the HMOs shall be used for human consumption. In addition, organic solvents are expensive to purchase and to dispose. Also, organic solvents are harmful to the environment and to personnel handling them. Thus, crystallization of HMOs is a drawback in the production of HMOs in an industrial scale and should be avoided, especially at the end of the recovery process of the desired HMO.

Document EP 2 896 628 A1 pertains to a process for efficient purification of neutral human milk oligosaccharides from fermentation broths. The process involves a combination of a cation exchanger treatment, and anion exchanger treatment, and a nanofiltration and/or electrodialysis step. The HMO may be obtained in solid from by spray-drying, as crystalline material or as sterile filtered concentrate.

Laid open publication WO 2015/049331 A1 discloses a process for purifying a neutral HMO utilizing simulated moving bed chromatography. This process allows a continuous purification of a neutral HMO from a crude solution to obtain the HMO in high purity in solid form by spray-drying or as a concentrated syrup.

Document US 2015/183814 A1 discloses a method for enhancing the stability of a human milk oligosaccharide of a HMO blend when stored for extended periods at temperatures above 25 °C. The method involves spray-drying an aqueous solution of the HMO or HMO blend to remove at least 90 % of the water. This document also discloses a method for removing organic solvent residues from a HMO or a HMO blend by spray-drying, and a method for enhancing the bioavailability of a HMO of HMO blend by spray-drying.

Documents EP 3 486 326 A1, EP 3 494 804 A1, EP 3 494 805 A1, EP 3 494 806 A1, and EP 3 494 807 A1 were published after the filing date of the instant patent, and each document discloses a nutritional composition comprising a mixture consisting of

| saccharide | [wt.-%] |
|---|---|
| 2'-fucosylactose (2'-FL) | 34 |
| 3-fucosyllactose (3-FL) | 11 |
| lacto-*N*-tetraose (LNT) | 20 |
| lacto-*N*-neotetraose (L*N*nT) | 2 |
| lacto-N-fucopentaose I (L*N*FPI) | 13 |
| 3'-sialyllactose (3'-SL) | 3 |
| 6'-sialyllactose (6'-SL) | 4 |
| N-acetylneuraminic acid (NeuAc) | 8 |
| L-fucose | 5 |
| Total | 100 |

Röhrig and colleagues (Röhrig, C. et al. (2004) Critical Reviews in Food Science and Nutrition 57 (5) 1017-1038) summarize on the biochemistry, metabolism, bioavailability and data on N-acetylneuraminic acid and N-glycolyl-D-neuraminic acid levels that occur in diverse foods. It is disclosed that the human body is able to synthesize sialic acid endogenously, and that sialic acid occurs in breast milk in free form - although predominantly present in bound form as outermost sugar of oligosaccharides and glycoconjugates. It is also mentioned that human milk contains L-fucose and N-acetyl-D-glucosamine as monosaccharides. In addition to the utilization of endogenously produced sialic acid, the human body is able to utilize sialic acid from dietary sources such as breast milk or of animal origin. The latter contains significant levels of the unfavorable N-glycolyl-D-neuraminic acid which has been identified as a risk factor for long-term adverse health effects. Röhrig et al. disclose that extremely high purity NeuAc can only be obtained from alternative manufacturing processes if robust crystallization procedures are applied that provide a crystalline ingredient.

Therefore, there is a need for processes which provides a mixture of HMOs, preferably in combination with at least one monosaccharide, in a solid form, wherein the process is applicable in industrial scale production and does not involve the use of an organic solvent at the end of the purification of one or more of the HMOs to provide a solid preparation of said HMOs.

The object has been achieved by a process for the manufacture of a spray-dried powder comprising a mixture of structurally distinct HMOs.

## Summary

In a first aspect, a spray-dried powder containing a mixture of structurally distinct HMOs and at least one monosaccharide selected from the group consisting of L-fucose and N-acetylneuraminic acid is provided, as defined in claim 1.

In a second aspect, the use of the spray-dried powder containing a mixture of structurally distinct HMOs and at least one monosaccharide selected from the group consisting of L-fucose and N-acetylneuraminic acid for the manufacture of a nutritional composition is provided, as defined in claim 6.

The invention is set out in the appended set of claims.

## Description of the figures

Fig. 1 shows a graph illustrating the results of a X-ray powder diffraction of spray-dried 3-fucosyllactose.

Fig. 2 shows a graph illustrating the results of a X-ray powder diffraction of spray-dried lacto-*N*-tetraose.

Fig. 3 shows a graph illustrating the results of a X-ray powder diffraction of spray-dried 6'-sialyllactose.

Fig. 4 shows a graph illustrating the results of a X-ray powder diffraction of spray-dried 3'-sialyllactose.

Fig. 5 shows a graph illustrating the results of a X-ray powder diffraction of a spray-dried mixture of 2'-fucosyllactose and lacto-*N*-tetraose.

Fig. 6 shows a graph illustrating the results of a X-ray powder diffraction of a spray-dried mixture of 2'-fucosyllactose, 3-fucosyllactose, lacto-*N*-tetraose, 3'-sialyllactose, and 6'-sialyllactose.

## Detailed description

According to the first aspect, a spray-dried powder is provided which contains a mixture of structurally distinct HMOs, as defined in claim 1.

The spray-dried powder preferably contains at least 80 %-wt., at least 85 %-wt., at least 90 %-wt., at least 93 %-wt., at least 95 %-wt. or at least 98 %-wt. HMOs..

The spray-dried powder contains a mixture of structurally distinct HMOs and at least one monosaccharide selected from the group consisting of L-fucose and N-acetylneuraminic acid (NeuAc). In an additional and/or alternative embodiment, the spray-dried powder contains in addition to the mixture of structurally distinct HMOs the monosaccharides L-fucose and *N*-acetylneuraminic acid.

The spray-dried powder comprises the composition provided in Table 2, with the exception of a composition comprising

Table 2: Composition of the spray-dried powder

| saccharide | [wt.-%] |
|---|---|
| 2'-fucosylactose (2'-FL) | 34 |
| 3-fucosyllactose (3-FL) | 11 |
| lacto-*N*-tetraose (L*N*T) | 20 |
| lacto-*N*-neotetraose (L*N*nT) | 2 |
| lacto-N-fucopentaose I (L*N*FPI) | 13 |
| 3'-sialyllactose (3'-SL) | 3 |
| 6'-sialyllactose (6'-SL) | 4 |
| N-acetylneuraminic acid (NeuAc) | 8 |
| L-fucose | 5 |
| Total | 100 |
| 2'-FL | 30.0 - 55.0 |
| 3-FL | 10.0 - 15.0 |
| L*N*T | 20.0 - 30.0 |
| L*N*nT | 0.0 - 5.0 |

(continued)

| saccharide | [wt.-%] |
|---|---|
| L*N*FPI | 0.0 - 20.0 |
| 3'-SL | 2.0 - 4.0 |
| 6'-SL | 4.0 - 6.0 |
| NeuAc | 0.0 - 10.0 |
| L-fucose | 0.0 - 6.0 |
| Total | 100.0 |

In an additional and/or alternative embodiment, at least one of the HMOs of the mixture of structurally distinct HMOs and/or of the spray-dried powder has been produced by microbial fermentation. In a particular embodiment all HMOs of the mixture of structurally distinct HMOs and/or of the spray-dried powder have been produced by microbial fermentation.

In an additional and/or alternative embodiment, said at least one monosaccharide has been produced by microbial fermentation. In another embodiment wherein the spray-dried powder contains L-fucose and N-acetylneuraminic acid, both monosaccharides have been produced by microbial fermentation.

Hence, in a particular embodiment, all saccharides that are present in the spray-dried powder, i.e. the HMOs and the monosaccharides, have been produced by microbial fermentation.

In an additional and/or alternative embodiment, at least one of the HMOs in the spray-dried powder, preferably all HMOs in the spray-dried powder, are present in amorphous form. In certain embodiments, the monosaccharide, at least one of the monosaccharides or both monosaccharides is/are present in amorphous form.

In an additional and/or alternative embodiment, the spray-dried powder contains a low amount of water. The term "low amount of water" refers to an amount of $\leq$ 15 %-wt. of water, preferably $\leq$ 10 %-wt. of water, more preferably $\leq$ 7 %-wt. of water, most preferably $\leq$ 5 %-wt. of water.

In an additional and/or alternative embodiment, the spray-dried powder is free of genetically-engineered microorganisms and free of nucleic acid molecules derived from genetically-engineered microorganisms.

A spray-dried powder containing a mixture of structurally distinct HMOs in combination with at least one monosaccharide selected from the group consisting of L-fucose and N-acetylneuraminic acid, is advantageous over liquid compositions in that said spray-dried powder is less vulnerable to microbial contamination. Said spray-dried powder is also advantageous over powders having the same composition of ingredients which were obtained by freeze-drying or lyophilization in that the spray-dried powder is less hygroscopic and remains flowable much longer.

Provided, but not falling under the scope of the present invention, is also a process for the manufacture of the spray-dried powder comprising a mixture of structurally distinct HMOs and at least one monosaccharide selected from the group consisting of L-fucose and N-acetylneuraminic acid, wherein at least one of the structurally distinct HMOs, preferably all of the structurally distinct HMOs has/have been produced by microbial fermentation. The process comprises the steps of:

a) recovering at least one of the structurally distinct HMOs from a fermentation broth;
b) providing an aqueous solution of the at least one HMO of step a); and
c) subjecting the solution of step b) to spray-drying.

In an additional and/or alternative embodiment, the purification of the at least one HMO from fermentation broth (step a) includes one or more of the steps of

i) removing microbial cells from the fermentation broth to obtain a process stream;
ii) subjecting the process stream to at least one ultrafiltration;
iii) treating the process stream at least one time with a cation exchange resin and/or at least one time with an anion exchange resin;
iv) subjecting the process stream to at least one nanofiltration;
v) subjecting the process stream to at least one electrodialysis;
vi) treating the process stream at least one time with activated charcoal; and/or
vii) subjecting the process stream at least one time to a crystallization and/or precipitation step.

The at least one HMO or any one of the structurally distinct HMOs of the mixture may be produced by microbial

fermentation, wherein a genetically-engineered microorganism that is able to synthesize an HMO is cultivated in a culture medium (fermentation broth) and under conditions that are permissive for the synthesis of said HMO by said genetically-engineered microorganism. The purification of the HMO that was synthesized by the cells of the genetically-engineered microorgansim comprises the step of separating the microbial cells from the fermentation broth to obtain a process stream. The process stream is essentially free of cells and contains said HMO(s). This step is the first step in the process of purifying the desired HMO.

Suitable methods for separating the microbial cells from the fermentation broth include centrifugation wherein the microbial cells are obtained as a pellet and the fermentation broth as a supernatant. In an additional and/or alternative embodiment, the microbial cells are separated from the fermentation broth by means of filtration. Suitable filtration methods for separating the cells from the fermentation broth include microfiltration and ultrafiltration.

Microfiltration as such is a physical filtration process where a particle-containing fluid is passed through a special pore-sized membrane to separate the particles from the fluid. The term "microfiltration" as used herein referst to a physical filtration process where cells are seprated from the fermentation broth.

Ultrafiltration is a variety of membrane filtration and is not fundamentally different. In ultrafiltration, forces like pressure or concentration gradients lead to a separation through a semipermeable membrane. Cells, suspended solids and solutes of high molecular weight are retained in the so-called retentate, while water and low molecular weight solutes such as the desired sialylated oligosaccharide pass through the membrane in the permeate (filtrate).

Ultrafiltration membranes are defined by the molecular weight cut-off (MWCO) of the membrane used. Ultrafiltration is applied in cross-flow or dead-end mode.

Typically, the microbial cells synthesize the HMOs intracellularly. Depending on the structure of the HMO, the HMO is either delivered into the fermentation broth or remains inside the microbal cell. In the former case, the thus produced HMO is present in the fermentation broth at the end of the fermentation, and can be recovered from the fermentation broth which thus becomes the process stream. In the latter case, the microbial cells bearing the HMOs are separated from the fermentation broth and are lysed to release the HMOs. Thus, the cell lysate containd the HMO and becomes the process stream for the purification of the HMO as described herein after.

Notwithstanding that the process is used for the purification of HMOs that have been produced by microbial fermentation, said process may also be employed to purify HMOS that were produced by enzymatic catalysis *in-vitro.* The HMO can then be purified from the reaction mixture at the end of the biocatalytic reaction. Said reaction mixture is subjected to the process for the purification as process stream.

The process stream contains the desired HMO(s) as well as by-products and undesired impurities such as - for example - monosaccharides, disaccharides, undesired oligosaccharide by-products, ions, amino acids, polypeptides, proteins and/or nucleic acid molecules.

In an additional and/or alternative embodiment, the process for the purification of the HMO comprises the step of at least one cation exchange treatment to remove positively charged compounds from the cleared process stream.

Suitable cation exchange resins for removing postively charged compounds from the process stream include Lewatit® S 6368 A (Lanxess AG, Cologne, DE) in $H^+$ form; Lewatit® S 2568 ($H^+$) (Lanxess AG, Cologne, DE).

In an additional and/or alternative embodiment, the process for the purification of the HMO comprises the step of an anion exchange treatment to remove undesired negatively charged compounds from the cleared process stream.

Suitable anion exchange resins include Lewatit® S 2568 ($Cl^-$) (Lanxess AG, Cologne, DE) Lewatit® S 6368 A (Lanxess AG, Cologne, DE), Lewatit® S 4268 (Lanxess AG, Cologne, DE), Lewatit® S 5528 (Lanxess AG, Cologne, DE), Dowex® AG 1x2 (Mesh 200-400), Dowex® 1x8 (Mesh 100-200), Purolite® Chromalite® CGA100x4 (Purolite GmbH, Ratingen, DE), Dow® Amberlite™ FPA51 (Dow Chemicals, MI, USA).

In as additional/or alternative embodyment, the process for the purification of the HMO comprises a nanofiltration and/or a diafiltration step to remove impurities having a lower molecular weight, and to concentrate the desired HMO.

Diafiltration involves the addition of fresh water to a solution in order to remove (wash out) membrane-permeable components. Diafiltration can be used to separate components on the basis of their molecular size and charge by using appropriate membranes, wherein one or more species are efficiently retained and other species are membrane permeable. In particular, diafiltration using a nanofiltration membrane is effective for the separation of low molecular weight compounds like small molecule and salts. Nanofiltration membranes usually have a molecular weight cut-off in the range 150 - 1000 Daltons. Nanofiltration is widely used in the dairy industry for the concentration and demineralization of whey.

Suitable membranes for nanofiltration and/or diafiltration include Dow® Filmtec™ NF270-4040, Trisep® 4040-XN45-TSF (Microdyn-Nadir GmbH, Wiesbaden, DE), GE4040F30 and GH4040F50 (GE Water & Process Technologies, Ratingen, DE).

Diafiltration using nanofiltration membranes was found to be efficient as a pretreatment to remove significant amounts of contaminants prior to electrodialysis treatment of the solution containing the oligosaccharide. The use of nanofiltration membranes for concentration and diafiltration during the purification of HMOs results in lower energy and processing costs, and better product quality due to reduced thermal exposure, leading to reduced Maillard reactions and aldol reactions.

In an additional and/or alternative embodiment, the process for the purification of the HMO comprises at least one electrodialysis step.

Electrodialysis (ED) combines dialysis and electrolysis and can be used for the separation or concentration of ions in solutions based on their selective electromigration through semipermeable membranes.

The basic principle of electrodialysis consists of an electrolytic cell comprising a pair of electrodes submerged into an electrolyte for the conduction of ions, connected to a direct current generator. The electrode connected to the positive pole of the direct current generator is the anode, and the electrode connected to the negative pole is the cathode. The electrolyte solution then supports the current flow, which results from the movement of negative and positive ions towards the anode and cathode, respectively. The membranes used for electrodialysis are essentially sheets of porous ion-exchange resins with negative or positive charge groups, and are therefore described as cationic or anionic membranes, respectively. The ion-exchange membranes are usually made of polystyrene carrying a suitable functional group (such as sulfonic acid for cationic membranes or a quaternary ammonium group for anionic membranes) cross-linked with divinylbenzene. The electrolyte can be, for example, sodium chloride, sodium acetate, sodium propionate or sulfamic acid. The electrodialysis stack is then assembled in such a way that the anionic and cationic membranes are parallel as in a filter press between two electrode blocks, such that the stream undergoing ion depletion is well separated from the stream undergoing ion enrichment (the two solutions are also referred to as the diluate (undergoing ion depletion) and concentrate (undergoing ion enrichment). The heart of the electrodialysis process is the membrane stack, which consists of several anion-exchange membranes and cation-exchange membranes separated by spacers, installed between two electrodes. By applying a direct electric current, anions and cations will migrate across the membranes towards the electrodes.

In an additional and/or alternative embodiment, the process for the purification of the HMO further comprises a step of continuous chromatography like simulated bed moving (SMB) chromatography.

Simulated moving bed (SMB) chromatography originated in the petrochemical and mineral industries. Today, SMB chromatography is used by the pharmaceutical industry to isolate enantiomers from racemic mixtures. Large-scale SMB chromatography has already been used for the separation of the monosaccharide fructose from fructose-glucose solutions and for the separation of the disaccharide sucrose from sugar beet or sugar cane syrups.

SMB chromatography processes used to separate saccharides use e.g. calcium charged, cross-linked polystyrene resins, anion resins in the bisulfite form (Bechthold M., et al., Chemie Ingenieur Technik, 2010, 82, 65-75), or polystyrenic gel strong acid cation resin in the hydrogen form (Purolite® PCR833H) (Purolite, Bala Cynwyd, USA).

Given the continuous mode of operation, the recycling of the mobile phase and also the potential to use large column sizes, SMB chromatography systems can in principle be scaled to achieve production volumes of hundreds of tons.

The process step of simulated moving bed chromatography is advantageous in that this process step allows further removal of oligosaccharides being structurally closely related to the desired oligosaccharide.

In an additional and/or alternative embodiment, the process for the purification of the at least one HMO comprises a treatment of the process stream with activated charcoal to remove contaminating substances such as colorants from the process stream.

In additional and/or alternative embodiment, the process for the purification of the at least one HMO comprises at least one step of crystallization or precipitation of the HMO from the process stream. Crystallization or precipitation of the at least one HMO from the process stream may be performed by adding a suitable amount of an organic solvent that is miscible with water to the process stream containing the at least one HMO. The organic solvent may be selected from the group consisting of $C_1$- to $C_6$-alcohols and $C_1$- to $C_4$-carboxylic acids. The step of crystallization or precipitation of the at least one HMO is not performed at the end of the recovery process such that residual amounts of the organic solvent or carboxylic acid can be removed by subsequent process steps.

In an additional and/or alternative embodiment of the process for the purification of the at least one HMO comprises a step sterile filtration and/or endotoxin removal, preferably by filtration of the process stream through a 3 kDa filter or 6 kDa filter.

In an additional and/or alternative embodiment, the process for the purification of the at least one HMO comprises a step of increasing the concentration of the at least one HMO in the process stream. The concentration of the at least one HMO in the process stream can be increased by subjecting the process stream to vacuum evaporation, reverse osmosis or nanofiltration (e.g. nanofiltration with a nanofiltration membrane having a size exclusion limit of ≤ 20 Å). Alternatively, crystallized or precipitated HMOs are dissolved in water to obtain an aqueous solution of the at least one HMO, wherein the aqueous solution possessing the desired concentration of said at least one HMO.

In an additional and/or alternative embodiment, the resulting process stream is an aqueous solution which contains the at least one HMO in a concentration of ≥ 20 g/l, ≥ 25 g/l, ≥ 30 g/l, ≥ 40 g/l, ≥ 60 g/l, ≥ 100 g/l, ≥ 200 g/l or even ≥ 300 g/l.

In an additional and/or alternative embodiment, the aqueous solution contains the at least one HMO in a purity of at least 80 %, at least 85 %, at least 90 %, at least 93 %, at least 95 % or at least 98 % with respect to the weight of dry

matter/solutes within the solution.

The term "purity" as used herein refers to chemical purity, i.e. the degree to which a substance is undiluted or unmixed with extraneous material. Hence, the chemical purity is an indicator of the relationship between the at least one HMO and by-products/impurities. Chemical purity is expressed as a percentage (%) and is calculated using the following formula:

$$\text{Percent Purity} = 100 \times \frac{\text{Mass of desired compound in sample}}{\text{Total mass of sample}}$$

The purity of an HMO in a preparation can be determined by any suitable method known to the skilled artisan, for example by using HPLC and calculating the ratio of the area underneath the peak(s) representing the amount of HMO(s) to the sum of areas underneath the peaks representing the HMO(s) and all other compounds than said HMO(s) in the same chromatogram.

The aqueous solution containing the at least one HMO can be stored under appropriate conditions, for example said aqueous solution can be frozen.

The process for the purification of the at least one HMO is cost efficient and easy to scale up, making it suitable as a basis for a multi-ton scale manufacturing process.

The process for the purification of the at least one HMO is also advantageous in that the aqueous solution is free of genetically-engineered microorganisms and nucleic acid molecules derived from genetically-engineered microorganisms. In addition, the aqueous solution is free of proteins. The total removal of proteins eliminates the risk of causing allergies to a potential consumer.

The process for the manufacture of the spray-dried powder comprises the step of providing an aqueous solution containing the at least one HMO or the mixture of structurally distinct HMOs, and - optionally - the at least one monosaccharide.

In an additional and/or alternative embodiment, the at least one HMO is selected from the group consisting of 2'-FL, 3-FL, LNT, LNnT, LNFPI, 3'-SL, 6'-SL.

In an additional and/or alternative embodiment, the mixture of structurally distinct HMOs consists of five structurally distinct HMOs, preferably of 2'-FL, 3-FL, LNT, 3'-SL and 6'-SL. In another embodiment, the mixture of structurally distinct HMOs consists of seven structurally distinct HMOs, preferably of 2'-FL, 3-FL, LNT, LNnT, LNFPI, 3'-SL and 6'-SL.

In an additional and/or alternative embodiment, the aqueous solution further contains at least one monosaccharide, preferably selected from the group consisting of L-fucose and N-acetylneuraminic acid. In another embodiment, the aqueous solution further contains L-fucose and N-acetylneuraminic acid.

In an additional and/or alternative embodiment, the aqueous solution contains the at least one HMO or the mixture of HMOs, and/or the at least one monosaccharide, in an amount totaling a saccharide amount of at least 20 % (w/v), 30 % (w/v), 35 % (w/v), and up to 45 % (w/v), 50 % (w/v), 60 % (w/v).

In an additional and/or alternative embodiment, the aqueous solution contains the at least one HMO or the mixture of HMOs in a purity of at least 80 %, at least 85 %, at least 90 %, at least 93 %, at least 95 % or at least 98 % with respect to the weight of dry matter/solutes within the solution.

In an additional and/or alternative embodiment, the aqueous solution does not contain genetically-engineered microorganisms, nucleic acid molecules derived from genetically-engineered microorganisms and proteins.

In the process for the manufacture of the spray-dried powder, the aqueous solution containing the at least one HMO or the mixture of structurally distinct HMOs is subjected to spray-drying.

Spray-drying is a method to obtain dry powders, wherein the solution containing the substance of interest is first sprayed into droplets which are rapidly dried by hot air. Spray-drying is very fast and exposure of the substance to be dried to high temperatures is quite short.

In an additional and/or alternative embodiment, the aqueous solution containing the at least one HMO or the mixture of structurally distinct HMOs is spray-dried at a nozzle temperature of at least 110 °C, preferably at least 120 °C, more preferably at least 125 °C, and less than 150 °C, preferably less than 140 °C and more preferably less than 135 °C.

In an additional and/or alternative embodiment, the aqueous solution containing the at least one HMO or the mixture of structurally distinct HMOs is spray-dried at an outlet temperature of at least 60 °C, preferably at least 65 °C, and less than 80 °C, preferably less than 70 °C. In a particularly preferred embodiment, the aqueous solution containing the HMO(s) is spray-dried at a nozzle temperature of about 68 °C to about 70 °C.

It is understood that each species of the structurally distinct HMOs - optionally in combination with the at least one monosaccharide - can be purified and spray-dried individually, and that the resulting spray-dried powders can be mixed in any desired ratio. In an alternative embodiment, the aqueous solutions contains all structural distinct HMOs, and the resulting aqueous solution containing the mixture of structurally distinct HMOs in the desired ratio is subjected to spray-drying.

In an additional and/or alternative embodiment, the aqueous solution containing at least one HMO or a mixture of structurally distinct HMOs further contains at least one monosaccharide selected from the group consisting of L-fucose and N-acetylneuraminic acid. The aqueous solution containing the at least one HMO or the mixture of structurally distinct HMOs, and the at least one monosaccharide is then subjected to spray-drying to obtain a spray-dried powder consisting essentially of the at least one HMO and the at least one monosaccharide or the mixture of structurally distinct HMOs and the at least one monosaccharide.

The ratio of saccharides (HMOs and monosaccharide(s)) in the resulting spray-dried powder corresponds to the ratio of these saccharides in the aqueous solution.

The latter procedure is advantageous in that monosaccharides which can not be spray-dried individually become spray-dryable in the presence of one or more HMOs.

The spray-drying of the aqueous solution containing the at least one HMO or the mixture of structurally distinct HMOs provides a powder of low hygroscopy, wherein the HMO is present in amorphous form, and wherein the particle size is homogeneous. The spray-dried powder containing the mixture of structurally distinct HMOs is less hygroscopic than a powder of identical composition which was obtained by freeze-drying. Thus, the spray-dried powders as described herein are advantageous with respect to their further use and processing.

According to the second aspect, provided is the use of the spray-dried powder containing a mixture of structurally distinct HMOs and at least one monosaccharide selected from the group consisting of L-fucose and N-acetylneuraminic acid, for the manufacture of a nutritional composition. The spray-dried powder is suitable for human consumptions and may thus be included into preparations for human consumption such as medicinal formulations, infant formula, dairy drinks or dietary supplements.

The nutritional composition contains one or more additional ingredients. Said one or more additional ingredients are selected from the group consisting of oil, fat and fatty acids (such as olive oil, sunflower oil, coconut oil, nut oil, rapeseed oil, palm oil, flaxseed oil, fish oil, linolenic acid, soy-bean oil, etc.), carbohydrates (such as glucose, fructose, lactose, maltodextrin, starch, sucrose, inositol, etc.) proteins (from skim milk, whey, casein (derived from any domesticated dairy animals), or soy bean), vitamins (A, B1, B2, B5, B6, B12,C, D, E, K, biotin, folic acid, niacin, choline) minerals and trace elements (sodium, potassium, chloride, calcium, phosphorus, magnesium, iron, zinc, manganese, fluoride, selenium, iodine, copper).

The nutritional composition containing the spray-dried powder containing the mixture of human milk oligosaccharides and the at least one monosaccharide may be an infant formula that meets the compositional requirements set forth in Regulation (EU) 2016/127 and/or in the Code of Federal Regulations (USA) Title 21 107.100 (nutrient specifications). Representative compositions of infant formulas are specified in Table 3.

Table 3: Composition of a representative infant formula. The final concentration is based on a preparation of 13.5 g of the powder in 90 ml of water.

| | | | per 100 g powder | per 100 ml infant formula |
|---|---|---|---|---|
| Energy | | kJ | 2094-2145 | 283 |
| | | kcal | 500-512 | 67-68 |
| Fats, hereof: | | g | 24.2-26.2 | 3.3-3.5 |
| | saturated fatty acids | g | 8.7-9.4 | 1.2-1.3 |
| | monounsaturated fatty acids | g | 10.4 | 1.4 |
| | polyunsaturated fatty acids | g | 5.5-5.9 | 0.7-0.8 |
| Carbohydrates hereof: | | g | 56-58 | 7.4-7.9 |
| | | | | |
| | Sugars | g | 44-56 | 6-7.4 |
| | hereof: | | | |
| | Lactose | g | 44-56 | 6-7.4 |
| | Neu5Ac | mg | 440 | 60 |
| | L-fucose | mg | 300 | 40 |
| | HMOs Hereof | g | 4.22-4.81 | 0.57-0.65 |
| | 2'-FL | g | 1.85-2.22 | 0.25-0.30 |

(continued)

| | | per 100 g powder | per 100 ml infant formula |
|---|---|---|---|
| 3-FL | mg | 555.56-592.6 | 75-80 |
| L*N*T | g | 1.11 | 0.15 |
| L*N*nT | mg | 0-111.11 | 0-15 |
| L*N*PF-I | mg | 0-740.74 | 0-100 |
| 3'-SL | mg | 148.15-170.37 | 20-23 |
| 6'-SL | mg | 207.4-222.22 | 28-30 |
| Protein | 9 | 11.11-11.85 | 1.5-1.6 |
| Salt | 9 | 0.47-0.59 | 0.06-0.08 |
| Vitamins | | | |
| Vitamin A | µg | 357-358 | 47.3-48.2 |
| Vitamin D | µg | 7.8 | 1.05 |
| Vitamin E | mg | 8.15 | 1.1 |
| Vitamin K | µg | 43.7-44.4 | 5.9-6.0 |
| Vitamin C | mg | 115-118 | 15-16 |
| Vitamin B1 | mg | 0.51-0.60 | 0.068-0.079 |
| Vitamin B2 | mg | 1.3-1.7 | 0.18-0.23 |
| Niacin | mg | 3.63 | 0.49 |
| Vitamin B6 | µg | 526-600 | 71-81 |
| Folic acid | µg | 160-164 | 21.6-21.7 |
| Vitamin B12 | µg | 1.7-1.9 | 0.23-0.25 |
| Biotin | µg | 22-30 | 3.0-3.9 |
| Panthothenic acid | mg | 4.6-5.4 | 0.62-0.72 |
| Minerals | | | |
| Sodium | mg | 187-236 | 25.3-31.2 |
| Potassium | mg | 673-675 | 88.8-91.2 |
| Chloride | mg | 327-333 | 43.1-44.9 |
| Calcium | mg | 460-504 | 62.1-66.5 |
| Phosphorous | mg | 335-352 | 45.2-46.5 |
| Magnesium | mg | 49.3-56.3 | 6.66-7.43 |
| Iron | mg | 4.15 | 0.56 |
| Zinc | mg | 3.7-3.8 | 0.49-0.51 |
| Copper | µg | 274 | 37 |
| Manganese | µg | 96.3 | 13 |
| Fluoride | µg | 30.4-32.6 | 4.1-4.4 |
| Selenium | µg | 11.1-12.3 | 1.5-1.6 |
| Iodine | µg | 101.5-103.7 | 13.7-14 |

In an additional and/or alternative embodiment, the nutritional composition also contains microorganisms, preferably probiotic microorganisms. For infant food applications, the preferred microorganisms are derived from or can be found in the microbiome of a healthy human. Preferably, but with no limitations, the microorganisms are selected from the

genera *Bifidobacterium, Lactobacillus, Enterococcus, Streptococcus, Staphylococcus, Peptostreptococcus, Leuconostoc, Clostridium, Eubacterium, Veilonella, Fusobacterium, Bacterioides, Prevotella, Escherichia, Propionibacterium* and *Saccharomyces.* In an additional and/or alternative embodiment, the microorganism is selected from the group consisting of *Bifidobacterium adolescentis, B. animalis, B. bifidum, B. breve, B. infantis, B. lactis, B. longum; Enterococcus faecium; Escherichia coli; Klyveromyces marxianus; Lactobacillus acidophilus, L. bulgaricus, L. casei, L. crispatus, L. fermentum, L. gasseri, L. helveticus, L. johnsonii, L. paracasei, L. plantarum, L. reuteri, L. rhamnosus, L. salivarius, L. sakei; Lactococcus lactis* (including but not limited to the subspecies *lactis, cremoris* and *diacetylactis*); *Leuconostoc mesenteroides* (including but not limited to subspecies *mesenteroides*); *Pedicoccus acidilactici, P. pentosaceus; Propionibacterium acidipropionici, P. freudenreichii* ssp. *shermanii; Staphylococcus carnosus;* and *Streptococcus thermophilus.*

In addition to the combination living organisms, the nutritional composition can also include dead cell cultures. In the field of probiotics, killed cell cultures are sometimes used (e.g. tyndalized bacteria). These killed cultures may provide proteins, peptides, oligosaccharides, cell outer wall fragments and natural products, leading to the short-term stimulation of the immune system.

Including probiotic microorganisms in the nutritional composition, especially in the presence of HMOs, is particularly advantageous in that it also promotes the establishment of a healthy gut microbiome.

In an additional and/or alternative embodiment, the nutritional composition also includes prebiotics such as galacto-oligosaccharides (GOS), fructo-oligosaccharides (FOS), inulin or combinations thereof.

The nutritional composition may be present in liquid form or in solid form including, but not limited to, powders, granules, flakes and pellets.

In an additional embodiment, the nutritional composition is selected from the group consisting of medicinal formulations, infant formulas and dietary supplements.

The present invention will be described with respect to particular embodiments and with reference to drawings, but the invention is not limited thereto but only by the claims.

It is to be noticed that the term "comprising", as used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification do not necessarily refer always to the same embodiment. Furthermore, the particular features, structures or characteristics may be combined in any suitable manner, as would be apparent to one of ordinary skill in the art from this disclosure, in one or more embodiments.

Similarly, it should be appreciated that in the description of representative embodiments of the invention, various features of the invention are sometimes grouped together in a single embodiment, figure, or description thereof for the purpose of streamlining the disclosure and facilitating the understanding of one or more of the various inventive aspects. This method of disclosure is not to be interpreted as reflecting an intention that the claimed invention requires more features than are expressly listed in each claim. Rather, as the following claims reflect, inventive aspects may require fewer than all the features of any foregoing disclosed embodiment. Thus, the claims following the detailed description are hereby expressly incorporated into this detailed description, with each claim standing on its own as a separate embodiment of this invention.

In the description and drawings provided herein, numerous specific details are set forth. However, it is understood that embodiments of the invention may be practiced without these specific details. In other instances, well-known methods, structures and techniques have not been shown in detail in order to facilitate the understanding of the description and drawings.

Examples 1 to 7 as set forth herein below are not part of the claimed invention.

**Example 1: Purification of 2'-fucosyllactose from fermentation broth**

Production of 2'-fucosyllactose by fermentation using a genetically modified *E. coli* strain was performed as described in European patent application No. 16 196 486.1. The 2'-fucosyllactose was purified from the fermentation broth by filtration, ion exchange chromatography, nanofiltration, diafiltration or electrodialysis, and treatment with charcoal as described in WO 2015/106943 A1. The resulting solution containing 2'-fucosyllactose was subjected to spray-drying to obtain a stable solid product.

**Example 2: Purification of 3-fucosyllactose from fermentation broth**

3-Fucosyllactose was produced by fermentation using a genetically modified *E. coli* strain as described in European patent application No. 16 196 486.1.

The cells were separated from the culture medium by ultrafiltration (0.05 μm cut-off) (CUT membrane technology, Erkrath, Germany) followed by a cross-flow filter with a MWCO of 150 kDa (Microdyn-Nadir, Wiesbaden, Germany). The cell-free fermentation medium containing about 30 g/L 3-fucosyllactose, was passed over a strong cationic ion exchanger (Lewatit S 2568 (Lanxess, Cologne, Germany) in $H^+$ form to remove positive charged contaminants. Afterwards the solution was set to pH 7.0 using sodium hydroxide and applied to an anionic ion exchanger (Lewatit S6368 A, Lanxess) in the chloride form. Both ion exchangers were used in 200 L volume. After a second filtration (150 kDa; Microdyn-Nadir, Wiesbaden, Germany) the particle free solution was concentrated 5-fold by nanofiltration using a Filmtech NF270 membrane (Dow, Midland, USA) and 2.5-fold by vacuum evaporation. The concentrated solution and a conductivity of about 15 mS $cm^{-1}$ was filtrated (10 kDa; Microdyn-Nadir, Wiesbaden, Germany), clarified by activated carbon charcoal (CAS:7440-44-0, Carl Roth, Karlsruhe, Germany) and deionized by electrodialysis. Therefor a PC-Cell BED 1-3 electrodialysis apparatus (PC-Cell, Heusweiler, Germany) with a PC-Cell E200 membrane stack was used containing the following membranes: cation exchange membrane CEM:PC SK and anion membrane AEM:PCAcid60. 0.25 M Sulphamic acid was used as electrolyte in the process. For reduction of brownish coloring caused by Maillard-reactions and aldol products originating from the fermentation process, a second round of ion exchange chromatography was performed using the same ion exchange material as aforementioned in $Na^+$ and $Cl^-$ form, however in a volume of 50 L. After concentrating the sugar solution by evaporation, again the conductivity was reduced from 4 mS $cm^{-1}$ to 0.4 mS $cm^{-1}$ or less by electrodialysis using the PC-Cell BED 1-3 mentioned previously. For further decolorization the solution was mixed with activated charcoal (CAS:7440-44-0, Carl Roth, Karlsruhe, Germany) and a nearly colorless solution was obtained by filtration.

**Example 3: Purification of lacto-N-tetraose from fermentation broth**

Fermentative production of Lacto-N-tetraose was conducted using a genetically modified E. *coli* BL21 (DE3) Δ*lacZ* strain, with genomically integrated genes essential for the *in vivo* synthesis of Lacto-N-tetraose., namely, a N-acetylglucosamine glycosyltransferase (lgtA from *Neisseria meningitidis* MC58), a β-1,3-galactosyltransferases (*wbdO* from *Salmonella enterica* subsp. *salamae* serovar Greenside), lacY from E. *coli* K12, the UDP-glucose-4-epimerase galE, and the UTP-glucose-1-phosphat uridyltransferase galU, both from E. *coli* K12. For the fermentative production of Lacto-N-tetraose the strain was grown in a defined mineral salts medium, comprising 7 g $l^{-1}$ $NH_4H_2PO_4$, 7 g $l^{-1}$ $K_2HPO_4$, 2 g $l^{-1}$ KOH, 0.3g $l^{-1}$ citric acid, 5 g $l^{-1}$ $NH_4Cl$, 0.1 mM $CaCl_2$, 8 mM $MgSO_4$, trace-elements (0.101 g $l^{-1}$ nitrilotriacetic acid, pH 6.5, 0.056 g $l^{-1}$ ammonium ferric citrate, 0.01 g $l^{-1}$ $MnCl_2 \times 4\ H_2O$, 0.002 g $l^{-1}$ $CoCl_2 \times 6\ H_2O$, 0.001g $l^{-1}$ $CuCl_2 \times 2\ H_2O$, 0.002 g $l^{-1}$ boric acid, 0.009 g $l^{-1}$ $ZnSO_4 \times 7\ H_2O$, 0.001 g $l^{-1}$ $Na_2MoO_4 \times 2\ H_2O$, 0.002 g $l^{-1}$ $Na_2SeO_3$, 0.002 g $l^{-1}$ $NiSO_4 \times 6\ H_2O$) and 2% glucose as carbon source. Antifoam (Struktol J673, Schill + Seilacher) was added when needed. The pH was controlled using a 25% ammonia solution. Lactose was added stepwise to a final concentration of 15 mM from a 216 g $l^{-1}$ lactose stock, the lactose concentration in the culture medium was held constant throw-out the fermentation process. Residual lactose and Lacto-N-triose II, accumulation during the process as by-product, was hydrolyzed by a second E. *coli* strain that was added to the fermenter. This strain expressed a functional beta-lactamase, a beta-N-acetylhexosaminidase (*bbhI* from *Bifidobacterium bifidum* JCM1254), and a functional gal-operon for degradation of monosaccharides (EP 2 845 905 A).

Cells were separated from the fermentation broth, and the lacto-N-tetraose containing fluid was purified to a purity of 75-80 %, determined by mass balance, according to the procedure described in Example 2.

Contaminating carbohydrate by-products resulting from inefficient enzymatic degradation and metabolization were removed by chromatography using a simulated moving bed (SMB) chromatography, according to WO 2015/049331. Alternatively, the lacto-N-tetraose was purified by crystallization with isopropanol. For crystallization the lacto-N-tetraose containing solution was concentrated by evaporation to a concentration of 20% and spray-dried. Using a NUBILOSA LTC-GMP spray dryer (NUBILOSA, Konstanz, Germany) the solution was passed under nitrogen flow through the spray dryers nozzles with an inlet temperature of 130°C while the product flow was controlled to maintain an outlet temperature of 67°C to 68°C.

The solid material was added to a mixture of isopropanol and water (3:1 (vol/vol)) in a ratio of 1 kg powder in 12 L isopropanol/water. The suspension was stirred vigorously, then the insoluble lacto-N-tetraose was filtrated and dried at 40 °C. Starting with a 73-89 % pure material, the crystallized Lacto-N-tetraose was purified to about 95 %, with a recovery of 85 %. The sugar was dissolved in water to a concentration of 25 % and passed sequentially through a 6 kDa filter (Pall Microza ultrafiltration module SIP-2013, Pall Corporation, Dreieich, Germany) and a 0.2 μm sterile filter. Solid material was obtained by spray drying the sterile material under the conditions described above.

**Example 4: Purification of 3'- and 6'-Sialyllactose from fermentation broth**

For production of 3'- and 6'-sialyllactose recombinant E. *coli* BL21 (DE3) Δ*lacZ* strains were used. The strains had common genetic modifications: chromosomal, constitutive expression of the glucosamine-6-phosphate synthase GlmS from E. *coli,* the N-acetylglucosamin 2-epimerase Slr1975 from *Synechocystis sp.,* the glucosamine 6-phosphat N-acetyltransferase Gna1 from *Saccharomyces cerevisiae,* the phosphoenolpyruvat synthase PpsA from E. *coli,* the N-acetylneuraminate synthase NeuB, and the CMP-sialic acid synthetase NeuA, the latter both from *Campylo-bacterjejuni.* Additionally, the genes encoding the lactose permease LacY from E. *coli, cscB* (sucrose permease), cscK (fructokinase), *cscA* (sucrose hydrolase), and *cscR* (transcriptional regulator) from E. *coli* W, and a functional gal-operon, consisting of the genes *galE* (UDP-glucose-4-epimerase), *galT* (galactose-1-phosphate uridylyltransferase), galK (galactokinase), and *galM* (galactose-1-epimerase) from E. *coli* K12 were integrated into the genome of the BL21 strain, and constitutively expressed.

The strain synthesizing 3'-sialyllactose harbors the 3'-sialyltransferase gene from *Vibrio* sp. JT-FAJ-16, while the 6'-sialyllactose producing strain contains the alpha-2,6-sialyltransferase plsT6 from *Photobacterium leiognathi* JT-SHIZ-119.

The sialyllactose producing strains were grown in a defined mineral salts medium, comprising 7 g $l^{-1}$ $NH_4H_2PO_4$, 7 g $l^{-1}$ $K_2HPO_4$, 2 g $l^{-1}$ KOH, 0.3g $l^{-1}$ citric acid, 5 g $l^{-1}$ $NH_4Cl$, 1 ml $l^{-1}$ antifoam (Struktol J673, Schill + Seilacher), 0.1 mM $CaCl_2$, 8 mM $MgSO_4$, trace-elements and 2% sucrose as carbon source. The sucrose feed (500 g $l^{-1}$), fed in the fed-batch phase was supplemented with 8 mM $MgSO_4$ , 0.1 mM $CaCl_2$, trace elements, and 5 g $l^{-1}$ $NH_4Cl$.

Trace elements consisted of 0.101 g $l^{-1}$ nitrilotriacetic acid, pH 6.5, 0.056 g $l^{-1}$ ammonium ferric citrate, 0.01 g $l^{-1}$ $MnCl_2 \times 4\ H_2O$, 0.002 g $l^{-1}$ $CoCl_2 \times 6\ H_2O$, 0.001g $l^{-1}$ $CuCl_2 \times 2\ H_2O$, 0.002 g $l^{-1}$ boric acid, 0.009 g $l^{-1}$ $ZnSO_4 \times 7\ H_2O$, 0.001 g $l^{-1}$ $Na_2MoO_4 \times 2\ H_2O$, 0.002 g $l^{-1}$ $Na_2SeO_3$, 0.002 g $l^{-1}$ $NiSO_4 \times 6\ H_2O$.

For sialyllactose formation, a lactose feed of 216 g $l^{-1}$ was employed. The pH was controlled by using ammonia solution (25% v/v). Fed batch fermentation was conducted at 30°C under constant aeration and agitation. In order to remove residual lactose at the end of the fermentation, β-galactosidase was added to the fermentation vessel. The resulting monosaccharides were metabolized by the production strain.

The cell-free liquid was then deionized by ion exchange chromatography. First, cationic contaminants were removed on a strong cationic exchanger in a volume of 200 L (Lewatit® S 2568 (Lanxess, Cologne, Germany) in $H^+$ form. Using NaOH the pH of the obtained solution was set to 7.0. In a second step, anionic ions and undesired colorants were removed from the solution using the strong anionic exchanger Lewatit® S 6368 S (Lanxess, Cologne, Germany) in the chloride form.

The ion exchanger had a bed volume of 200 L. Using a second filtration step on the cross-flow filter (150 kDa cut-off) (Microdyn-Nadir, Wiesbaden, Germany), precipitates originating from acidifying the solution were removed. For concentration of the sugar, the solution was nanofiltrated on a Dow FILMTECH NF270-4040 (Inaqua, Mönchengladbach, Germany), or, alternatively on a Trisep 4040-XN45-TSF Membrane (0.5 kDa cut-off) (Microdyn-Nadir, Wiesbaden, Germany). Using the latter, the monosaccharide N-acetylglucosamine, originating from the fermentation process and contaminating the sialyllactose solution, was separated from the product. The concentrated sialyllactose solution was then treated with activated charcoal (CAS:7440-44-0, Carl Roth, Karlsruhe, Germany) to remove colorants such as Maillard reaction products and aldol reaction products. In order to separate the sialyllactose from by-products that originate from the fermentation process like sialic acid and *N*-acetylglucosamine, the solution was filtrated on with a 1 kDa cut-off membrane GE4040F30 (GE water & process technologies, Ratingen, Germany), and diafiltrated to a conductivity of 0.6 to 0.8 mS $cm^{-1}$. The diluted solution was concentrated on a rotary evaporator to a concentration of about 300 g $L^{-1}$. In a final chromatographic separation other contaminating sugars, like di-sialyllactose were removed. Therefor the concentrated solution was applied to a weak anion ion exchange resin in the acetate form (Amberlite FPA51, Dow Chemical, Michigan, USA). While the sialyllactose rarely binds to the resin, the di-sialyllactose is adsorbed. Thus, the sialyllactose is eluted with 10 mM ammoniumacetat, while the di-sialyllactose is eluted with 1 M ammoniumacetat. For removal of the ammoniumacetat, the sialyllactose was precipitated with a 10-fold excess of ethanol. The solid fraction was filtrated and dried.

The product was finalized by passing a 20% sialyllactose solution sequentially through a 6 kDa filter (Pall Microza ultrafiltration module SIP-2013, Pall Corporation, Dreieich, Germany) and a 0.2 μm sterile filter.

A part of the solution was spray dried using a Büchi spray dryer (Büchi Mini Spray Dryer B-290) (Büchi, Essen, Germany), applying the following parameters: Inlet-temperature: 130°C, Outlet temperature 67°C-71°C, gasflow 670 L/h, aspirator 100%.

The spray-dried 6'-sialyllactose had a purity of 91%, while the 3'-sialyllactose material had a purity of 93%.

**Example 5: Preparation of HMO mixtures**

Mixtures of different HMOs were prepared from solid products. Therefore the individual HMOs were spray-dried

and the powders obtained were mixed. HMO-Mix I contained 2'-fucosyllactose and lacto-N-tetraose in a ratio of 70 %-wt to 30 %-wt; HMO-Mix II contained 2'-fucosyllactose (52 %-wt), 3-fucosyllactose (13 %-wt), lacto-N-tetraose (26 %-wt), 3'-sialyllactose (4 %-wt), and 6'-sialyllactose (5 %wt). The mixed powders were dissolved in water to a solution containing 20 %-wt HMOs, and the resulting solution was spray-dried again using the Büchi spray dryer as described in Example 4.

Analysis of the resulting spray-dried powder revealed that it had the same composition with respect to the ratio of the different HMOs as the solution subjected to spray-drying.

**Example 6: Preparation of saccharide mixtures**

8 g of 2'-FL and 1 g of L-fucose were dissolved in 50 ml distilled water. The resulting solution was spray-dried using the Büchi spray dryer as described in example 4. A spray-dried powder was obtained that consisted essentially of 2'-FL and L-fucose, wherein the ratio of 2'-FL and L-fucose in the spray-dried powder was identical to the ratio of 2'-FL and L-fucose in the solution that was subjected to the spray-drying. Thus, L-fucose can be spray-dried in the presence of an HMO.

**Example 7: Characterisation of spray-dried human milk oligosaccharides**

*1. Differential scanning calorimetry (DSC)*

Using differential scanning calorimetry (DSC) on a Mettler Toledo 821e (Mettler Toledo, Giessen, Germany) thermal events of spray-dried human milk oligosaccharides, namely 3-fucosyllactose, 6'-sialyllactose, 3'-sialyllactose, lacto-N-tetraose, and spray-dried mixtures of human milk oligosaccharides, a mixture (HMO-Mix I) of 2'-fucosyllactose/lacto-*N*-tetraose, and a mixture (HMO Mix II) of 2'-fucosyllactose, 3-fucosyllactose, lacto-N-tetraose, 6'-sialyllactose, 3'-sialyllactose, respectively, were determined.

A Mettler Toledo 821e (Mettler Toledo, Giessen, Germany) was used to determine thermal events of the spray-dried products (glass transition temperature (Tg), further exo- and endothermic events).

Approximately 25 mg of the spray-dried human milk oligosaccharides were analyzed in crimped Al-crucibles (Mettler Toledo, Giessen, Germany). The samples were cooled to 0 °C with 10 K/min and reheated to 100 °C with a scanning rate of 10 K/min. After cooling down the samples to 0 °C in a second heating cycle, the samples were reheated to 150 °C. The midpoint of the endothermic shift of the baseline during the heating scan was taken as glass transition temperature (Tg). Exothermic and endothermic peaks are reported by means of the peak temperature and the normalized energy of the event.

The first heating scan in all samples showed a main glass transition event in the total heat flow, as evidenced by a main step transition in the range of approximately 48-58 °C, in most of the samples, the major glass transition event observed in the first heating scan reoccurred in the second heating scan. The results of the DSC analyses are summarized in Table 4.

Table 4: Thermal events of HMOs as determined by differential scanning calorimetry

| Sample | 1st heating scan | 2nd heating scan |
|---|---|---|
| | Tg [°C] | Tg [°C] |
| 3-fucosyllactose | 57,6 | 59,9 |
| Lacto-N-tetraose | 49,9 | 79,4 |
| 6'-sialyllactose | 47,6 | 49,6 |
| 3'-sialyllactose | 48,8 | 54,3 |
| 2'-fucosyllactose/lacto-*N*-tetraose | 56,3 | 59 |
| HMO Mix | 54,2 | 55,6 |

For 3-fucosyllactose an endothermal relaxation peak after Tg in the first heating scan was detected. For lacto-N-tetraose a much higher Tg of about 79 °C was detected in the 2nd heating scan compared to that of the other samples. This might be caused by an endothermal event during the first heating scan at about 89 °C (-6.04 J/g). Like for 3-fucosyllactose, also for 6'-sialyllactose an endothermal relaxation peak was detected after Tg, however, in this sample additionally an endothermal event occurred at 77°C (-0.22 J/g). No endothermal events were detected for the 3'-sialyllactose and the HMO-Mix I, for HMO-Mix II the endothermal event during the 1st heating scan was at 79°C (0.34 J/g).

*2. X-ray powder diffraction (XRD)*

Wide angle X-ray powder diffraction (XRD) was used to study the morphology of lyophilized products. The X-ray diffractometer Empyrean (Panalytical, Almelo, The Netherlands) equipped with a copper anode (45 kV, 40 mA, $K_{\alpha 1}$ emission at a wavelength of 0.154 nm) and a PIXcel3D detector was used. Approximately 100 mg the spray-dried samples were analyzed in reflection mode in the angular range from 5-45° 2θ, with a step size of 0.04° 2θ and a counting time of 100 seconds per step.

All singular oligosaccharides as well as the HMO Mixes I and II showed a fully amorphous state (Figures 1 to 6). For lacto-N-tetraose a second (amorphous) signal was detected around 9-10°.

*3* Laser *diffraction*

The powder particle size was assessed by laser diffraction. The system detects scattered and diffracted light by an array of concentrically arranged sensor elements. The software-algorithm is then approximating the particle counts by calculating the z-values of the light intensity values, which arrive at the different sensor elements. The analysis was executed using a SALD-7500 Aggregate Sizer (Shimadzu Corporation, Kyoto, Japan) quantitative laser diffraction system (qLD).

A small amount (spatula tip) of each sample was dispersed in 2 ml isooctane and homogenized by ultrasonication for five minutes. The dispersion was transferred into a batch cell filled with isooctane and analyzed in manual mode.

Data acquisition settings were as follows: Signal Averaging Count per Measurement: 128, Signal Accumulation Count: 3, and Interval: 2 seconds.

Prior to measurement, the system was blanked with isooctane. Each sample dispersion was measured 3 times and the mean values and the standard deviation are reported. Data was evaluated using software WING SALD II version V3.1. Since the refractive index of the sample was unknown, the refractive index of sugar (disaccharide) particles (1.530) was used for determination of size distribution profiles. Size values for mean and median diameter are reported.

The mean particle sizes for all samples were very similar, slightly lower values were measured for HMO-Mix II. The particle size characteristics are summarized in Table 5. In addition, the particle size distribution showed the presence of one main size population for all of the samples.

Table 5: Particle size of HMOs as determined by laser diffraction

| Size | 3-FL | LNT | 6'-SL | 3'-SL | HMO-Mix I | HMO Mix II |
|---|---|---|---|---|---|---|
| Mean [nm] | 119.2 ± 0.5 | 117.3 ± 0.7 | 113.8 ± 1.5 | 115.4 ± 0.6 | 113.1 ± 0.3 | 97.3 ± 5.3 |
| Median [nm] | 141.3 ± 0.0 | 141.3 ± 0.0 | 141.3 ± 0.0 | 121.9 ± 16.7 | 141.3 ± 0.0 | 112.2 ± 0.0 |

## Claims

**1.** A spray-dried powder containing a mixture of structurally distinct human milk oligosaccharides and at least one monosaccharide selected from the group consisting of L-fucose and N-acetylneuraminic acid, wherein the spray-dried powder comprises

| saccharide | [wt.-%] |
|---|---|
| 2'-fucosylactose (2'-FL) | 30.0 - 55.0 |
| 3-fucosyllactose (3-FL) | 10.0 - 15.0 |
| lacto-*N*-tetraose (L*N*T) | 20.0 - 30.0 |
| lacto-*N*-neotetraose (L*N*nT) | 0.0 - 5.0 |
| lacto-N-fucopentaose I (L*N*FPI) | 0.0 - 20.0 |
| 3'-sialyllactose (3'-SL) | 2.0 - 4.0 |
| 6'-sialyllactose (6'-SL) | 4.0 - 6.0 |
| N-acetylneuraminic acid (NeuAc) | 0.0 - 10.0 |
| L-fucose | 0.0 - 6.0 |

(continued)

| saccharide | [wt.-%] |
|---|---|
| Total | 100.0 |

with the exception of a composition comprising

| saccharide | [wt.-%] |
|---|---|
| 2'-fucosylactose (2'-FL) | 34 |
| 3-fucosyllactose (3-FL) | 11 |
| lacto-*N*-tetraose (L*N*T) | 20 |
| lacto-*N*-neotetraose (L*N*nT) | 2 |
| lacto-N-fucopentaose I (L*N*FPI) | 13 |
| 3'-sialyllactose (3'-SL) | 3 |
| 6'-sialyllactose (6'-SL) | 4 |
| N-acetylneuraminic acid (NeuAc) | 8 |
| L-fucose | 5 |
| Total | 100 |

2. The spray-dried powder according to claim 1, wherein the spray-dried powder contains at least 80 %-wt., at least 85 %-wt., at least 90 %-wt., at least 93 %-wt., at least 95 %-wt. or at least 98 %-wt. human milk oligosaccharides.

3. The spray-dried powder according to claim 1 or 2, wherein at least one HMO of the mixture, preferably all HMOs of the mixture, are present in amorphous form.

4. The spray-dried powder according to any one of claims 1 to 3, wherein the at least one monosaccharide, preferably all monosaccharides, are present in amorphous form.

5. The spray-dried powder according to any one of claims 1 to 4, wherein the spray-dried powder contains ≤ 15 %-wt. of water, preferably ≤ 10 %-wt. of water, more preferably ≤ 7 %-wt. of water, most preferably ≤ 5 %-wt. of water.

6. Use of the spray-dried powder according to any one of claims 1 to 5 for the manufacture of a nutritional composition, preferably of an infant formula.

**Patentansprüche**

1. Sprühgetrocknetes Pulver, das eine Mischung aus strukturell unterschiedlichen humanen Milch-Oligosacchariden und zumindest ein Monosaccharid ausgewählt aus der Gruppe bestehend aus L-Fucose und N-Acetylneuraminsäure enthält, wobei das sprühgetrocknete Pulver Folgendes umfasst:

| Saccharid | [Gew.-%] |
|---|---|
| 2'-Fucosylactose (2'-FL) | 30,0-55,0 |
| 3-Fucosyllactose (3-FL) | 10,0-15,0 |
| Lacto-*N*-tetraose (L*N*T) | 20,0-30,0 |
| Lacto-*N*-neotetraose (L*N*nT) | 0,0-5,0 |
| Lacto-*N*-fucopentaose I (L*N*FPI) | 0,0-20,0 |
| 3'-Sialyllactose (3'-SL) | 2,0-4,0 |
| 6'-Sialyllactose (6'-SL) | 4,0-6,0 |

(continued)

| Saccharid | [Gew.-%] |
|---|---|
| N-Acetylneuraminsäure (NeuAc) | 0,0-10,0 |
| L-Fucose | 0,0-6,0 |
| Gesamt | 100,0 |

mit der Ausnahme einer Zusammensetzung, umfassend

| Saccharid | [Gew.-%] |
|---|---|
| 2'-Fucosylactose (2'-FL) | 34 |
| 3-Fucosyllactose (3-FL) | 11 |
| Lacto-*N*-tetraose (L*N*T) | 20 |
| Lacto-*N*-neotetraose (L*N*nT) | 2 |
| Lacto-N-fucopentaose I (L*N*FPI) | 13 |
| 3'-Sialyllactose (3'-SL) | 3 |
| 6'-Sialyllactose (6'-SL) | 4 |
| N-Acetylneuraminsäure (NeuAc) | 8 |
| L-Fucose | 5 |
| Gesamt | 100 |

**2.** Sprühgetrocknetes Pulver nach Anspruch 1, wobei das sprühgetrocknete Pulver zumindest 80 Gew.-%, zumindest 85 Gew.-%, zumindest 90 Gew.-%, zumindest 93 Gew.-%, zumindest 95 Gew.-% oder zumindest 98 Gew.-% humane Milch-Oligosaccharide enthält.

**3.** Sprühgetrocknetes Pulver nach Anspruch 1 oder 2, wobei zumindest ein HMO der Mischung, bevorzugt alle HMOs der Mischung, in amorpher Form vorhanden sind.

**4.** Sprühgetrocknetes Pulver nach einem der Ansprüche 1 bis 3, wobei das zumindest eine Monosaccharid, bevorzugt alle Monosaccharide, in amorpher Form vorhanden sind.

**5.** Sprühgetrocknetes Pulver nach einem der Ansprüche 1 bis 4, wobei das sprühgetrocknete Pulver ≤ 15 Gew.-% Wasser, bevorzugt ≤ 10 Gew.-% Wasser, bevorzugter ≤ 7 Gew.-% Wasser, am meisten bevorzugt ≤ 5 Gew.-% Wasser enthält.

**6.** Verwendung des sprühgetrockneten Pulvers nach einem der Ansprüche 1 bis 5 für die Herstellung einer Ernährungszusammensetzung, bevorzugt einer Säuglingsnahrung.

## Revendications

**1.** Poudre séchée par pulvérisation contenant un mélange d'oligosaccharides du lait maternel structurellement distincts et au moins un monosaccharide choisi dans le groupe constitué par le L-fucose et l'acide N-acétylneuraminique, dans laquelle la poudre séchée par pulvérisation comprend

| saccharide | [% en poids] |
|---|---|
| 2'-fucosylactose (2'-FL) | 30,0 à 55,0 |
| 3-fucosyllactose (3-FL) | 10,0 à 15,0 |
| lacto-*N*-tetraose (L*N*T) | 20,0 à 30,0 |

(continued)

| saccharide | [% en poids] |
|---|---|
| lacto-*N*-neotetraose (L*N*nT) | 0,0 à 5,0 |
| lacto-*N*-fucopentaose I (L*N*FPI) | 0,0 à 20,0 |
| 3'-sialyllactose (3'-SL) | 2,0 à 4,0 |
| 6'-sialyllactose (6'-SL) | 4,0 à 6,0 |
| acide N-acétylneuraminique (NeuAc) | 0,0 à 10,0 |
| L-fucose | 0,0 à 6,0 |
| Total | 100,0 |

à l'exception d'une composition comprenant

| saccharide | [% en poids] |
|---|---|
| 2'-fucosylactose (2'-FL) | 34 |
| 3-fucosyllactose (3-FL) | 11 |
| lacto-*N*-tetraose (L*N*T) | 20 |
| lacto-*N*-neotetraose (L*N*nT) | 2 |
| lacto-*N*-fucopentaose I (L*N*FPI) | 13 |
| 3'-sialyllactose (3'-SL) | 3 |
| 6'-sialyllactose (6'-SL) | 4 |
| acide N-acétylneuraminique (NeuAc) | 8 |
| L-fucose | 5 |
| Total | 100 |

**2.** Poudre séchée par pulvérisation selon la revendication 1, dans laquelle la poudre séchée par pulvérisation contient au moins 80 % en poids, au moins 85 % en poids, au moins 90 % en poids, au moins 93 % en poids, au moins 95 % en poids ou au moins 98 % en poids d'oligosaccharides du lait maternel.

**3.** Poudre séchée par pulvérisation selon la revendication 1 ou 2, dans laquelle au moins un HMO du mélange, de préférence tous les HMO du mélange, sont présents sous forme amorphe.

**4.** Poudre séchée par pulvérisation selon l'une quelconque des revendications 1 à 3, dans laquelle l'au moins un monosaccharide, de préférence tous les monosaccharides, sont présents sous forme amorphe.

**5.** Poudre séchée par pulvérisation selon l'une quelconque des revendications 1 à 4, dans laquelle la poudre séchée par pulvérisation contient $\leq$ 15 % en poids d'eau, de préférence $\leq$ 10 % en poids d'eau, plus préférablement $\leq$ 7 % en poids d'eau, le plus préférablement $\leq$ 5 % en poids d'eau.

**6.** Utilisation de la poudre séchée par pulvérisation selon l'une quelconque des revendications 1 à 5 pour la fabrication d'une composition nutritionnelle, de préférence d'une préparation pour nourrissons.

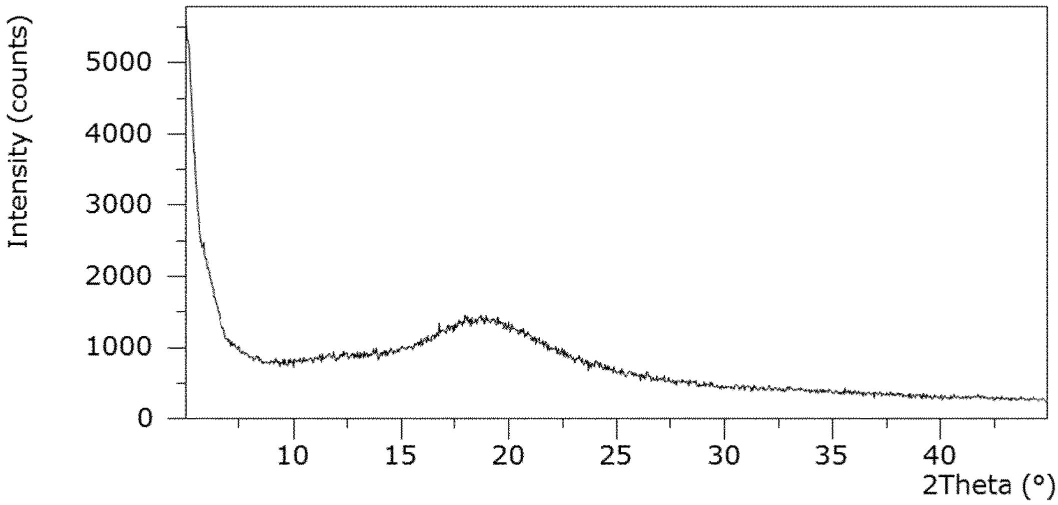
Intensity (counts)
5000
4000
3000
2000
1000
0
10
15
20
25
30
35
40
2Theta (°)

**FIG. 1**

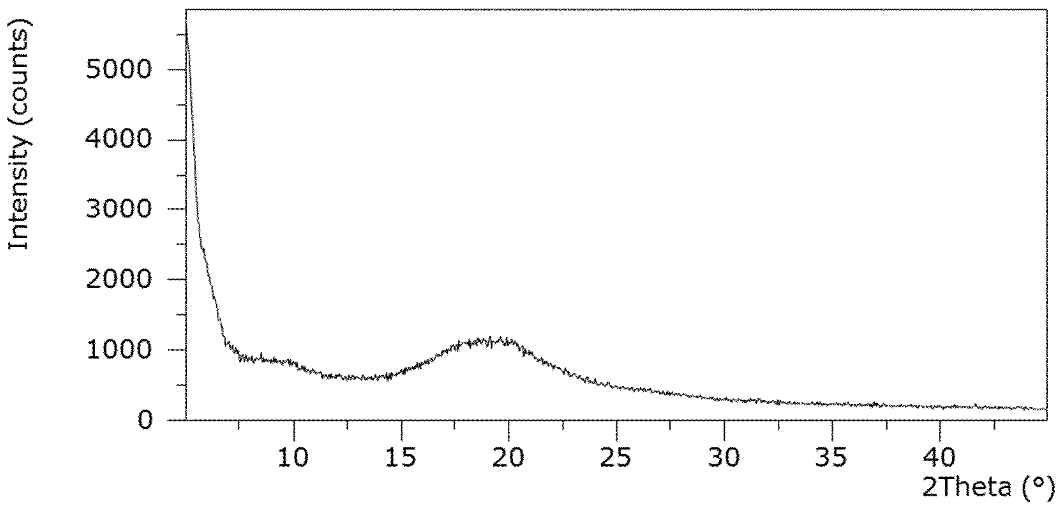
Intensity (counts)
5000
4000
3000
2000
1000
0
10
15
20
25
30
35
40
2Theta (°)

**FIG. 2**

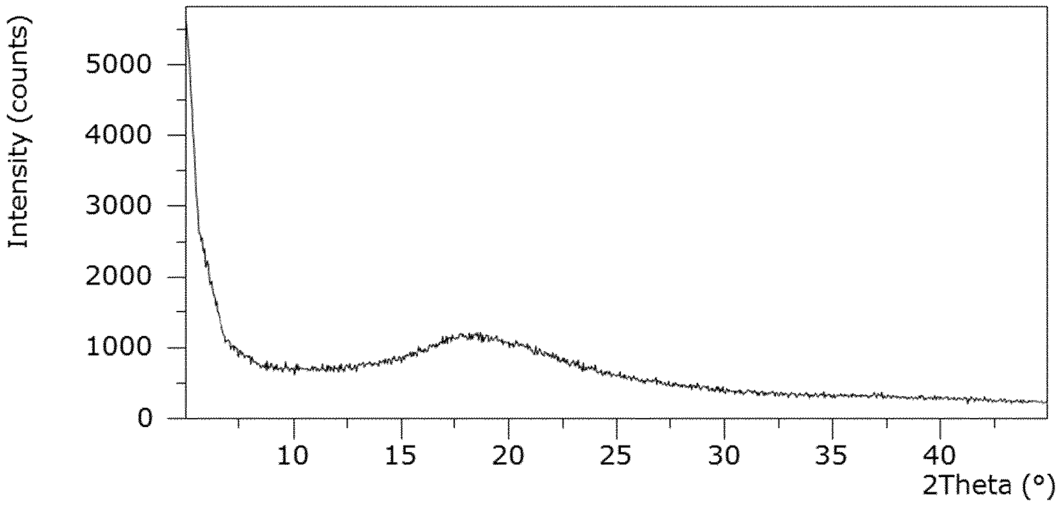
Intensity (counts)
5000
4000
3000
2000
1000
0
10
15
20
25
30
35
40
2Theta (°)

FIG. 3

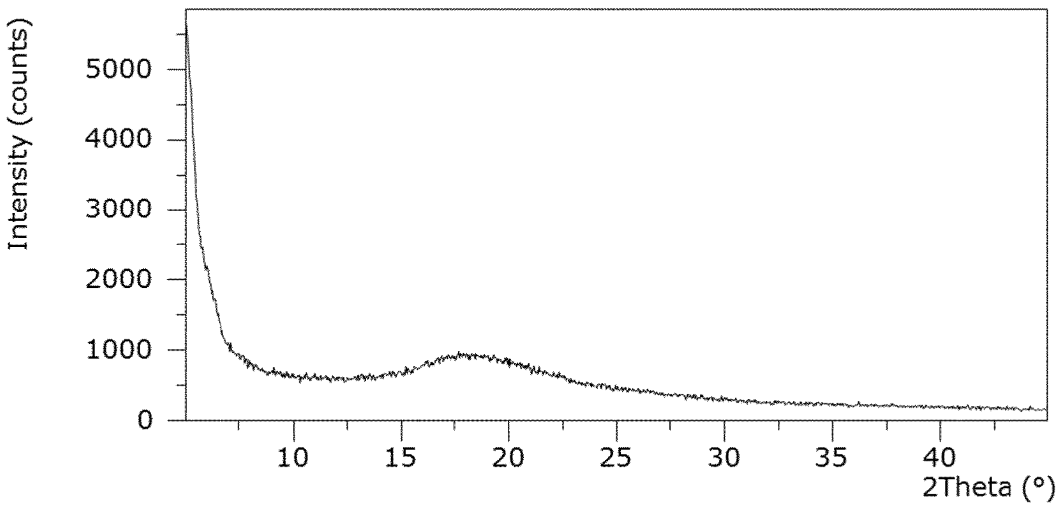
Intensity (counts)
5000
4000
3000
2000
1000
0
10
15
20
25
30
35
40
2Theta (°)

FIG. 4

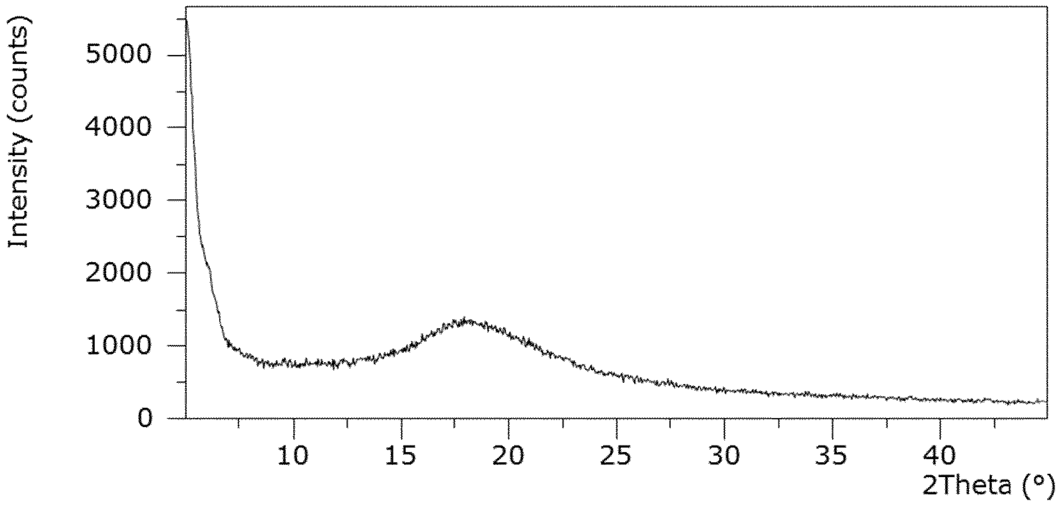
Intensity (counts)
5000
4000
3000
2000
1000
0
10
15
20
25
30
35
40
2Theta (°)

FIG. 5

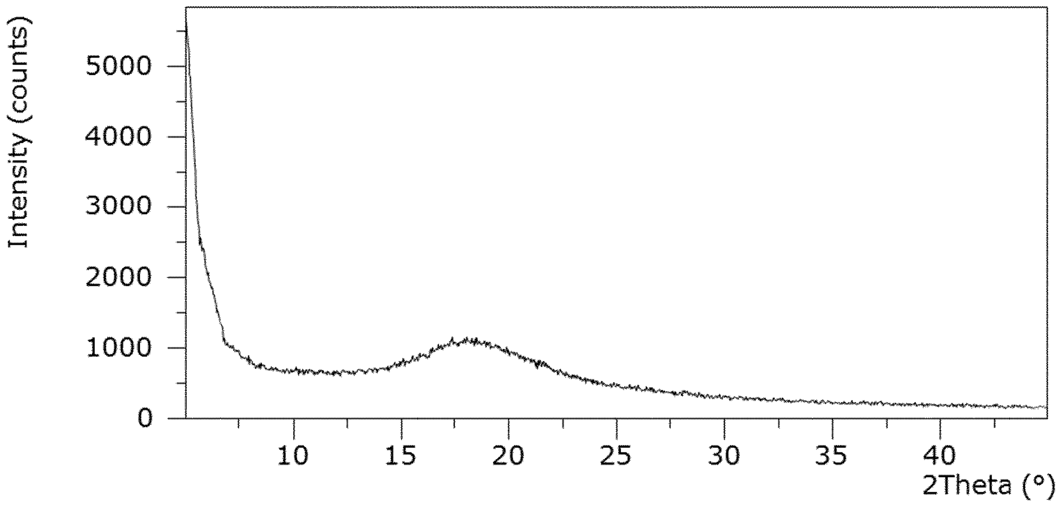
Intensity (counts)
5000
4000
3000
2000
1000
0
10
15
20
25
30
35
40
2Theta (°)

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015150328 A1
- WO 2017043382 A1
- WO 2010070104 A1
- WO 2012097950 A1
- WO 2014075680 A
- WO 2011150939 A
- WO 2016086947 A
- WO 2017101953 A
- WO 2014094783 A
- EP 2896628 A1
- WO 2015049331 A1
- US 2015183814 A1
- EP 3486326 A1
- EP 3494804 A1
- EP 3494805 A1
- EP 3494806 A1
- EP 3494807 A1
- EP 16196486 A
- WO 2015106943 A1
- EP 2845905 A
- WO 2015049331 A


### Non-patent literature cited in the description


- **THURL et al.** *Nutrition Reviews*, 2017, vol. 75 (11), 920-933
- Prebiotics and Probiotics in Human Milk, Origins and functions of milk-borne oligosaccharides and bacteria. Academic Press, 2017
- **RÖHRIG, C et al.** *Critical Reviews in Food Science and Nutrition*, 2004, vol. 57 (5), 1017-1038
- **BECHTHOLD M. et al.** *Chemie Ingenieur Technik*, 2010, vol. 82, 65-75
- *CHEMICAL ABSTRACTS*, 7440-44-0